Europäisches Patentamt

European Patent Office

Office européen des brevets

(19)

(11) Veröffentlichungsnummer: **0 275 604**
**B1**

(12)

# EUROPÄISCHE PATENTSCHRIFT

(45) Veröffentlichungstag der Patentschrift:
05.12.90

(51) Int. Cl.⁵: **E01B 9/40, E01B 9/68**

(21) Anmeldenummer: **87202596.0**

(22) Anmeldetag: **15.12.87**

(54) Unterlagsplatte für die Befestigung der Schienen von Eisenbahngleisen und -weichen auf Holzschwellen.

(30) Priorität: **20.12.86 DE 3643742**

(43) Veröffentlichungstag der Anmeldung:
**27.07.88 Patentblatt 88/30**

(45) Bekanntmachung des Hinweises auf die Patenterteilung:
**05.12.90 Patentblatt 90/49**

(84) Benannte Vertragsstaaten:
**AT BE CH DE ES FR GB IT LI LU NL SE**

(56) Entgegenhaltungen:
**CH-A- 216 352**
**US-A- 1 579 157**

(73) Patentinhaber: **Schwihag Gesellschaft für Eisenbahnoberbau mbH, Lebernstrasse 3, CH-8274 Tägerwilen(CH)**

(72) Erfinder: **Heim, Armin, Langhaldenstrasse 26, CH-8280 Kreuzlingen(CH)**

(74) Vertreter: **Müller, Gerd et al, Patentanwälte HEMMERICH-MÜLLER-GROSSE-POLLMEIER-MEY Hammerstrasse 2, D-5900 Siegen 1(DE)**

## Beschreibung

Die Erfindung betrifft eine Unterlagsplatte, insbesondere eine Rippenplatte, für die Befestigung der Schienen von Eisenbahngleisen und -weichen auf Holzschwellen oder anderem inhomogenen Material, die jeweils in der Nähe ihrer Enden mittels in Durchgangslöcher eingreifender Schrauben oder dergleichen mit der Schwelle verbunden ist, während im Plattenbereich zwischen den schwellenseitigen Befestigungsstellen jeweils der Schienenfuß abgestützt ist.

Die üblicherweise beim Bau von Eisenbahngleisen und -weichen zum Einsatz gelangenden Unterlagsplatten werden jeweils durch vier Schrauben oder dergleichen auf den Holzschwellen befestigt. Dabei wird eine Befestigung mit möglichst konstanten Einschraubkräften angestrebt. Diese Einschraubkräfte sollten für eine einzelne Schraube bis zu 35 kN betragen, können aber auch bis zu 50 kN erreichen. Daher kann die Unterlagsplatte durch diese hohen Einschraubkräfte einer Flächenlast von etwa 200 kN unterworfen werden.

Da die Befestigungsschrauben relativ nah an den Enden der Unterlagsplatte angreifen, stellt sich aufgrund der hohen Einschraubkräfte eine konkave Längsverformung der Unterlagsplatte ein, weil nämlich diese Unterlagsplatten nur eine meist auf 16 oder 20 mm begrenzte Dicke aufweisen und daher nur eine viel zu geringe Längssteifigkeit erreichen, die zu einer verformungsfreien Kraftübertragung nicht ausreicht.

Damit bspw. eine Norm-Rippenplatte URp 206, wie sie zur Zeit zur Befestigung von Schienen UIC 60 auf Holzschwellen benutzt wird, eine Längssteifigkeit erreicht, die eine praktisch verformungsfreie Einleitung der maximalen Flächenlast von 200 kN ermöglichen kann, müßte sie anstelle der normalen Dicke von 20 mm eine Dickenabmessung von etwa 35 mm erhalten, wenn eine einwandfrei gleichmäßig verteilte Flächenpressung auf die Holzschwellendecke erreicht werden soll.

Ein solchermaßen erhöhter Materialaufwand für die Rippenplatten ist jedoch im praktischen Einsatz wegen des hieraus resultierenden, erheblich höheren Kostenaufwandes untragbar. Auch wurde die Ursache der schon durch das Aufplatten entstehenden konkaven Wölbung bisher nicht erkannt.

Es wurde daher bisher in Kauf genommen, daß bei den üblichen Unterlagsplatten ständiger Berührungskontakt mit der Holzschwellendecke nur im Eingriffsbereich der Schwellenschraube vorhanden ist, während der mittlere Bereich jeder Unterlagsplatte mehr oder weniger konkav nach oben gewölbt wird, so daß sich dort, wo der Schienenfuß seine Auflagefläche hat, ein Hohlraum zur Holzschwellendecke hin bildet, über den hinweg die Unterlagsplatte sich beim Befahren durch die Wagenräder abwechselnd senkt und hebt -also sozusagen pumpt - wodurch nach einer gewissen Einsatzzeit die Oberfläche der Holzschwellendecke beschädigt oder sogar zerstört wird. Es lockert sich durch den Verschleiß die Unterlagsplatte, so daß die Spurweite nicht mehr eingehalten werden kann sowie die zwischen die Befestigungsschrauben und die Unterlagsplatte geschalteten Federringe brechen. Es kommt aber auch vor, daß aufgrund der ständigen Wechselbeanspruchungen die Unterlagsplatten selbst im kritischen Bereich brechen.

Dieser Mangel der bekannten Unterlagsplatten verursacht relativ hohe Unterhaltungskosten, weil die Holzschwellen wegen der Beschädigung der Schwellendecke oft ausgebaut und nach dem Abnehmen der Unterlagsplatten neu abgerichtet werden müssen. Es ist dann aber gleichzeitig notwendig, die bisher zur Aufnahme der Befestigungsschrauben vorgesehenen Löcher zu verdübeln und dann wieder neu zu bohren, damit dann die Befestigungsschrauben für die Unterlagsplatten wieder optimal befestigt werden können.

Da solche Regenerierungsarbeiten an einer Holzschwelle während ihrer Gesamtlebenszeit etwa zwei bis dreimal durchgeführt werden müssen, stellen sich bis zu deren biologischer Verrottung, also bis zum endgültigen Unbrauchbarwerden, beträchtliche Unterhaltungskosten ein.

Durch das Brechen der Federringe als Folge des Pumpvorganges wird alle 3 bis 4 Jahre ein Ersatzeinbau von neuen Federringen erforderlich, was ebenfalls hohe Kosten mit sich bringt. Durch die DE-OS 29 19 461 ist eine durch Walzen oder Pressen hergestellte, elastisch vorgespannte Unterlagsplatte bekanntgeworden, deren Ausgangsform konvex so vorgewölbt ist, daß sie nach der Befestigung auf der Holzschwellendecke nicht hiervon abhebt und daher auch beim Befahren durch die Wagenräder nicht pumpt. Durch diese Ausbildungs- und Funktionsform entfallen auch die sonst erforderlichen Federringe.

Bei dieser bekannten Unterlagsplatte hat die Auflagefläche einen immer unter einer Vorspannung stehenden Kontakt mit der Holzschwellendecke. Sie kann jedoch nur aus einem Werkstoff gefertigt werden, dessen Streckgrenze mindestens bei 520 N/mm² liegt.

Aus einem solchen, lediglich durch Walzen, Pressen und Gesenkschmieden zu bearbeitenden Material lassen sich daher nur einfache, unkomplizierte Bauformen von Unterlagsplatten relativ kostengünstig herstellen.

Für kompliziert gestaltete Unterlagsplatten, wie z.B. Gleitstuhl- und Stützbockplatten, die für den Bau von Weichen benötigt werden, ist die Herstellung durch Walzen, Pressen und Gesenkschmieden technisch nicht durchführbar.

Die komplizierten Unterlagsplatten, wie bspw. Gleitstuhl-und Stützbockplatten, lassen sich heute am wirtschaftlichsten durch Gießen herstellen, wobei meistens Sphäroguß GGG 40.3 zum Einsatz gelangt. Stahlguß kommt jedoch nicht in Frage; einerseits weil er zu teuer ist, andererseits aber auch weil er nicht mit genügend großer Maßgenauigkeit vergossen werden kann und daher eine kostenträchtige Nachbearbeitung erfordert.

Die Erfindung zielt darauf ab, die den bekannten Unterlagsplatten der gattungsgemäßen Art eigentümlichen Unzulänglichkeiten zu beseitigen. Deshalb liegt ihr die Aufgabe zugrunde, eine Unterlagsplatte der eingangs näher spezifizierten Art anzugeben, die mit einfachen Mitteln nach ihrer Befestigung mittels Schrauben oder dergleichen auf den Holz schwellen - dem sogenannten Aufplatten - eine ständig sichere und großflächigere Auflage als bisher auf der Schwellendecke gewährleistet, und das sogenannte Pumpen beim Befahren durch die Wagenräder eliminiert.

Gelöst wird diese Aufgabe erfindungsgemäß durch die im Kennzeichnungsteil des Anspruchs 1 angegebenen Merkmale, nämlich dadurch, daß an der Plattenunterseite lediglich im Bereich der Durchgangslöcher für die Befestigungsschrauben aus der Plattenebene vorspringende Podeste vorgesehen oder ausgebildet sind.

Beim Anziehen der die Durchgangslöcher durchsetzenden Befestigungsschrauben wird die Unterlagsplatte zunächst nur mit den Podesten gegen die Holzschwellendecke verspannt, wobei dann im Verlauf der sich ständig erhöhenden Einschraubkräfte eine Beanspruchung an der Unterlagsplatte entsteht, durch die sich eine konvexe Wölbung ihres Schienenauflagebereiches einstellt, durch welche dieser auf die Holzschwellendecke gepreßt wird und damit gegen das sogenannte Pumpen stabilisiert ist.

Die Erfindung sieht dabei nach Anspruch 2 vor, daß die Podeste einstückig an die Plattenunterseite angeformt sind.

Nach Anspruch 3 wird jedoch auch die Möglichkeit in Betracht gezogen, daß die Podeste durch Beilageteile aus Blech oder Kunststoff gebildet und an der Platte verklemmt oder verrastet sind.

In der Regel genügt es, wenn die Podeste nach dem Vorschlag des Anspruchs 4 eine Dicke von etwa 1 bis 1,5 mm aufweisen. Der hierdurch gegenüber der Holzschwellendecke zunächst geschaffene Freiraum für das Einsinken der Podeste ist völlig ausreichend, um nach dem Anziehen der Befestigungsschrauben die konvexe Wölbungsverspannung der Unterlagsplatten sicherzustellen.

Der erfindungsgemäße Lösungsvorschlag hat den besonderen Vorteil, daß die Unterlagsplatten, gleichgültig ob sie eine einfache oder eine komplizierte Bauform aufweisen, aus jedem geeigneten Werkstoff und in der üblichen Art und Weise gefertigt werden können.

Als zweckmäßig erweisen sich Unterlagsplatten, bei denen erfindungsgemäß nach Anspruch 5 jedem Durchgangsloch sein eigener Podestbereich zugeordnet ist und bei denen die benachbarten Podestbereiche durch eine Längsnut voneinander getrennt sind. Vorzugsweise kann dabei der Abstand des Begrenzungsrandes der Podeste vom Begrenzungsrand des zugehörigen Durchgangsloches größer als der Durchmesser des Durchgangsloches gewählt werden.

Dabei ist es jedoch wichtig, daß zur Errichtung eines konvex wirkenden Momentes auf die Unterlagsplatte beim Aufplatten gemäß Anspruch 6 der Abstand des Durchgangsloch-Begrenzungsrandes vom benachbarten Platten-Ende und der zugehörigen, äußeren Podest-Querkante größer bemessen ist als der Abstand des Durchgangsloch-Begrenzungsrandes von der inneren Querkante des betreffenden Podestes. Die beim Anziehen der Befestigungsschrauben auftretenden Momente begünstigen dann nämlich die konvexe Wölbung des Schienenauflagebereiches der Unterlagsplatte und damit das optimale Aufpressen desselben auf die Holzschwellendecke. Hierdurch wird die jeweilige Schiene - im Gegensatz zu den bisherigen bekannten Lösungen - stabil abgestützt.

Besonders bewährt hat sich nach Anspruch 7 eine Ausbildung, wonach das Abstandsverhältnis der äußeren und der inneren Podest-Querkanten gegenüber dem Durchgangsloch-Begrenzungsrand bei etwa 1,2 : 1 liegt, so daß also der Abstand der äußeren Podest-Querkanten von der Achse des Durchgangsloches etwa 20% größer ausfällt als der Abstand der inneren Podest-Querkante von der Achse des Durchgangsloches.

Ein optimales Verformungsverhalten der Unterlagsplatten wird dann erreicht, wenn nach dem Vorschlag des Anspruchs 8 erfindungsgemäß die Podeste eine polygonförmige, z.B. etwa rechteckige oder quadratische Umrißform aufweisen, weil hierdurch das Verformungsverhalten der Unterlagsplatten ebenfalls günstig beeinflußt wird.

Die Erfindung schlägt nach Anspruch 9 des weiteren vor, daß in der Plattenunterseite jeweils an die inneren Podest-Längskanten und -Querkanten angrenzende Nuten vorgesehen sind. Auch scheint es vorteilhaft, im Bereich der beiden Bohrungen entsprechende Ringnuten vorzusehen, in die die Auswölbungen der Podestbleche eingreifen.

Nach einem anderen Ausbildungsvorschlag der Erfindung ist durch Anspruch 10 in Vorschlag gebracht, daß die die Podeste bildenden Beilageteile aus ihrer Ebene ausgeklinkte und hakenartig hochgestellte Klemm- oder Rastzungen aufweisen, die mit in der Plattenunterseite vorgesehenen Vertiefungen in Ausricht- und Halteingriff bringbar sind. Dabei können gemäß Anspruch 11 die die Podeste bildenden Beilageteile sich über die ganze Plattenbreite erstrecken, wobei die Klemm- oder Rastzungen sowie ein zusätzlich zwischen diesen vorgesehener Ausschnitt sich etwa auf halber Länge der Beilageteile befinden.

Um im Querschnitt kein zusätzliches Verbiegungsmoment zu erzeugen, wird auf der Längsmittellinie der Platte im Bereich der Podeste eine Aussparung bzw. Nut derart vorgesehen, daß die Resultierenden aus der Schwellenschraubenkraft mit der Resultierenden aus der Flächenpressung in einer Ebene liegen. Bisher wurden die Platten zusätzlich in der Mitte konkav quer gewölbt.

In der Zeichnung ist der Gegenstand der Erfindung an Ausführungsbeispielen dargestellt. Dabei zeigen

Figur 1 in der Draufsicht eine als gegossene Rippengleitstuhlplatte ausgeführte Unterlagsplatte mit angegossenen Podesten

Figur 2 die Rippengleitstuhlplatte nach Fig. 1 in Pfeilrichtung II gesehen sowie teilweise im Längsschnitt entlang den Linien A-B sowie C-D in Fig. 1

Figur 3 einen Schnitt entlang der Linie E-F in Fig. 1,

Figur 4 eine der Fig. 2 entsprechende Darstellung der Rippengleitstuhlplatte, wobei die beim Anziehen der Befestigungsschrauben auftretenden Kräfte und Momente dargestellt sind und auch die aus der durch die beim Anziehen der Befestigungsschrauben entstehende exakt definierte Flächenpressung hervorgebrachte Resultierende angedeutet ist,

Figur 5 eine der Fig. 3 entsprechende Darstellung, wobei die durch das Anziehen der Befestigungsschrauben auftretenden Kräfte und die Resultierende aus der Flächenpressungen angedeutet sind,

Figur 6 eine der Fig. 1 entsprechende Draufsichtdarstellung einer abgewandelten Bauart für eine Rippengleitstuhlplatte,.

Figur 7 eine Ansicht der Rippengleitstuhlplatte in Pfeilrichtung 7 der Fig. 6, wobei diese jeweils in den Bereichen G-H und I-K der Fig. teilweise geschnitten gezeigt ist, bei der die Podeste aus untergelegten Blechen oder Kunststoffteilen bestehen.

Figur 8 einen Schnitt entlang der Linie L-M-N-O in Fig. 6,

Figur 9 eine der Fig. 7 entsprechende Darstellung, wobei jedoch die beim Anziehen der Befestigungsschrauben auftretenden Kräfte und Momente sowie auch die Resultierende aus der Flächenpressung angedeutet sind und

Figur 10 einen der Fig. 8 entsprechenden Schnitt mit Andeutung der durch das Anziehen der Befestigungsschrauben auftretenden Kräfte sowie der Resultierenden aus den Flächenpressungen, die beide in einer Ebene liegen und dadurch ein Moment vermeiden, das zur Verbiegung in Querrichtung führt.

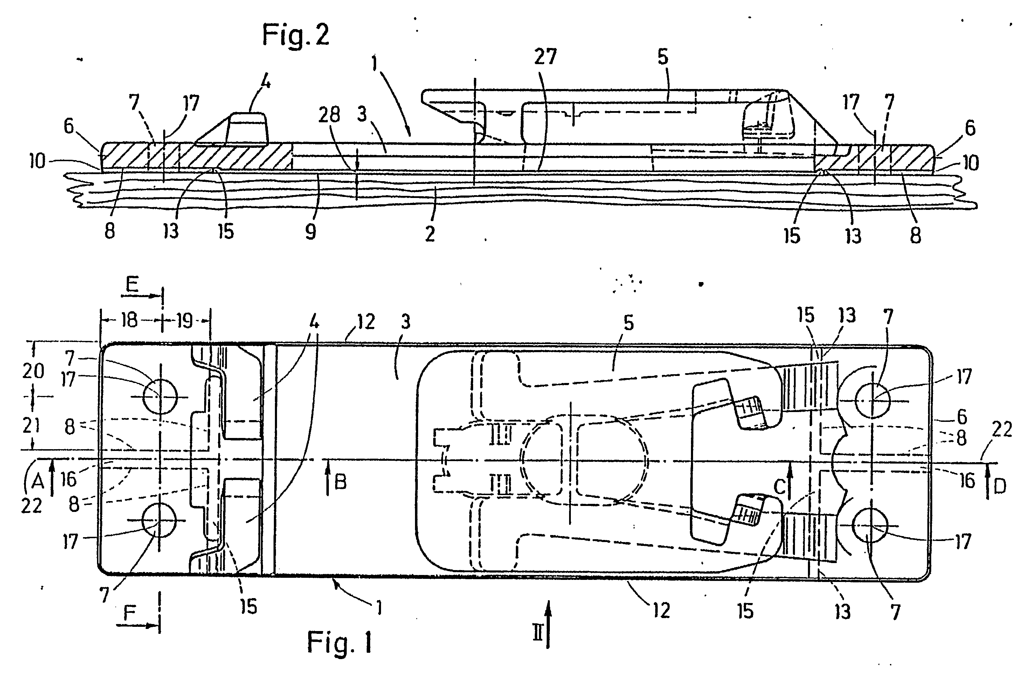

In den Fig. 1 bis 5 der Zeichnung ist als Anwendungsbeispiel der Erfindung eine gegossene Rippengleitstuhlplatte 1 gezeigt, wie sie zum Bau von Eisenbahnweichen in Verbindung mit Holzschwellen 2 benutzt wird.

Die Rippengleitstuhlplatte 1 weist dabei eine relativ lange und schmale Unterlagsplatte 3 auf, die auf ihrer Oberseite einstückig fest einerseits die Rippen 4 und andererseits den Gleitstuhl 5 trägt.

An ihren beiden Enden, also in der Nähe ihrer Querkanten 6 ist die Unterlagsplatte 3 jeweils mit zwei Durchgangslöchern 7 versehen, die zur Aufnahme der - nicht gezeigten - Befestigungsschrauben zur Bildung der schwellenseitigen Befestigungsstellen dienen.

Im Bereich zwischen den Durchgangslöchern 7 für die Befestigungsschrauben bildet einerseits die Unterlagsplatte 3 selbst die Stützauflage für den Schienenfuß einer - nicht gezeigten - festen Schiene, während andererseits der Gleitstuhl 5 als Stützauflage für den Schienenfuß einer -ebenfalls nicht gezeigten - Weichenzunge dient.

An ihrer Unterseite ist die Unterlagsplatte 3 lediglich im Bereich der Durchgangslöcher 7 für die Befestigungsschrauben mit aus der Plattenebene vorspringenden Podesten 8 ausge stattet, die eine relativ geringe Höhe, bspw. zwischen 1,0 und 2,0 mm aufweisen, und welche auf der Schwellendeckfläche 9 zur Stützauflage kommen.

Bei der Rippengleitstuhlplatte 1 nach den Fig. 1 bis 5 sind die Podeste 8 einstückig fest an die Unterseite der Unterlagsplatte 3 angeformt, und zwar dergestalt, daß jedem Durchgangslochbereich 7 ein eigener Podestbereich 8 zugeordnet ist. Dabei haben diese Podeste 8 eine polygonförmige, z.B. eine etwa quadratische Umrißform. Die äußere Querkante 10 der Podeste 8 hat dabei etwa Deckungslage mit der äußeren Querkante 6 der Unterlagsplatte 3, während ihre äußere Längskante etwa mit der Längskante 12 der Unterlagsplatte 3 zusammenfällt.

Die innere Querkante 13 jedes Podestes 8 wird durch eine in der Unterseite der Unterlagsplatte 3 befindliche Quernut 15 begrenzt, während die inneren Längskanten 14 derselben jeweils an eine in der Unterseite der Unterlagsplatte 3 befindliche - auf der Längsmitte 22-22 der Unterlagsplatte 3 liegende - Längsnut 16 angrenzen.

Jedes Podest 8 hat zum zugehörigen Durchgangslochbereich 7 der Unterlagsplatte 3 eine solche Relativlage, daß ihre äußere Querkante 10 von der Achse 17 dieses Durchgangsloches 7 einen Abstand 18 aufweist, welcher größer ist als der Abstand 19, den die innere Querkante 13 von dieser Achse 17 hat.

Der Abstand 20 der äußeren Längskante 11 jedes Podestes 8 von der Achse 17 des zugehörigen Durchgangsloches 7 sollte zweckmäßig das gleiche Maß haben wie der Abstand 21 der inneren Längskante 14 von dieser Achse 17.

Bewährt hat es sich im praktischen Einsatz, wenn das Verhältnis der Abstände 18 und 19 der äußeren und inneren Podest-Querkanten 10 und 13 relativ zur Längsachse 17 des Durchgangsloches 7 bei etwa 1,2 : 1 liegt, so daß der Abstand des Durchgangsloch-Begrenzungsrandes von der äußeren Querkante 10 um 20% größer ausfällt, als der Abstand der inneren Begrenzungskante 13 von diesem Durchgangsloch-Begrenzungsrand.

Bei der Anbringung der Rippengleitstuhlplatte 1 an der Holzschwelle 2 legt sich die Unterlagsplatte 3 zunächst lediglich mit den unteren Endflächen ihrer Podeste 8 auf die Schwellendeckfläche 9 der Holzschwelle 2 auf, wie das die Fig. 2 deutlich macht. Der die Rippen 4 und den Gleitstuhl 5 tragende Bereich der Unterlagsplatte 3 befindet sich jedoch von der Schwellendeckfläche 9 auf Distanz 28, wie das die Fig. 2 ebenfalls erkennen läßt.

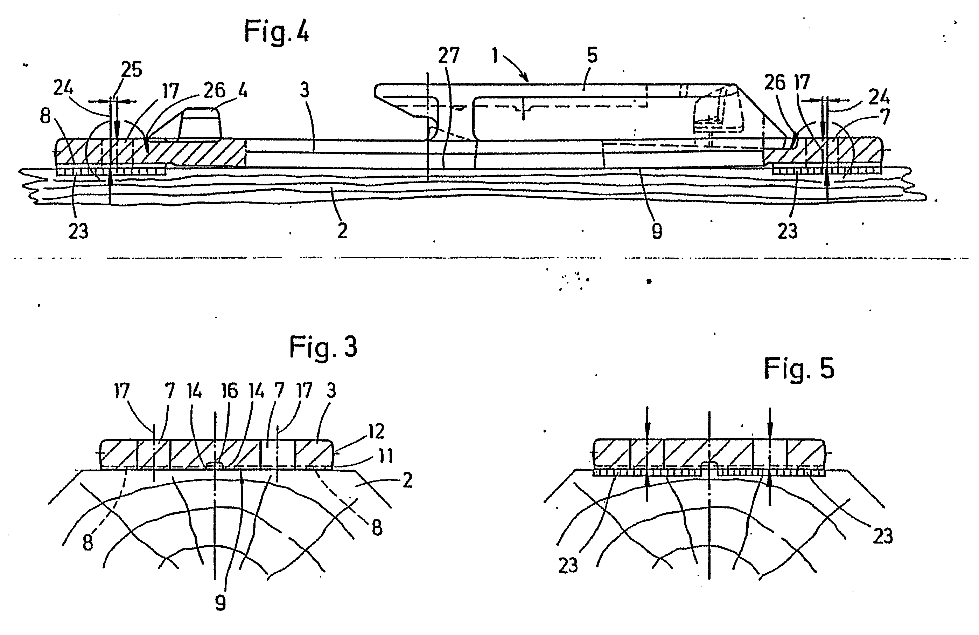

Sobald die - nicht gezeigten - Befestigungsschrauben im Bereich der Durchgangslöcher 7 in die Holzschwelle 2 eingedreht und bis zur Erreichung der maximal möglichen Einschraubkraft von 50 kN pro Befestigungsschraube angezogen werden, stellt sich zwischen der unteren Endfläche der Podeste 8 und der Schwellendeckfläche 9 der Holzschwelle 2 eine entsprechende Flächenpressung ein, die in den Fig. 4 und 5 der Zeichnung durch die jeweils mit senkrechter Schraffur versehenen Flächenpressungs-Felder 23 angedeutet ist.

Da die Resultierende aus der Flächenpressung 24 jedes Podestbereiches 8 relativ zur Achse 17 des zugehörigen Durchgangsloches 7 jeweils eine Versetztlage 25 aufweist, welche durch die Differenz der beiden Abstände 18 und 19 bestimmt wird, stellt sich durch das Anziehen der Befestigungsschraube im Bereich jedes Podestbereichs 8 ein Drehmoment ein, welches in Richtung des Pfeiles 26 auf die Unterlagsplatte 3 einwirkt und diese dadurch konvex nach unten bis zum Kontakt mit der Holzschwellendecke durchbiegt bzw. verformt, wie dies in Fig. 4 zu sehen ist. Hierdurch wird die Distanz 22 zwischen der Unterseite 27 der Unterlagsplatte 3 und der Schwellendeckfläche 9 überwunden, d.h., die Unterseite 27 der Unterlagsplatte 3 kommt auf die Schwellendeckfläche 9 zur Stützauflage, wie das der Fig. 4 entnommen werden kann.

Das die konvexe Verformung der Unterlagsplatte 3 bewirkende, durch die Befestigungsschrauben hervorgebrachte Verbiegungsmoment ist also abhängig von der Versetztlage 25 zwischen der Resultierenden der Flächenpressung 24 der Podeste und der Schwellenschraubenachse 17 des zugehörigen Durchgangsloches 7 für diese Befestigungsschraube. Die durch dieses Verbiegungsmoment herbeigeführte konvexe Verspannung der Unterlagsplatte 3 beruht darauf, daß die Podeste 8 im Bereich ihrer inneren Querkante 13 etwas tiefer in die Schwellendeckfläche 9 der Holzschwelle 2 einsinken können als deren äußere Querkanten. Durch die konvexe Durchbiegung bzw. Verformung der Unterlagsplatte 3 und die hieraus resultierende Stützauflage ihrer Unterseite 27 auf der Schwellendeckfläche 9 der Holzschwelle 2 wird erreicht, daß im kritischen Bereich der Rippengleitstuhlplatte keine Wechselbeanspruchung beim Befahren durch ein Schienenfahrzeug entsteht, sondern lediglich eine schwellende Beanspruchung auftritt. Der sogenannte Pumpvorgang der Rippengleitstuhlplatte 1 relativ zur Holzschwelle 2 wird damit vollkommen eliminiert. Die Schwellendeckfläche 9 der Holzschwelle wird geschont, so daß die Lebensdauer der letzteren gegen mechanische Zerstörung ein Mehrfaches der bisherigen Lebensdauer erhöht wird. Die ständige Überwachung gegen Lockerung und Bruch der Rippengleitstuhlplatten 1 entfällt und die hieraus resultierenden Unterhaltungsarbeiten werden vermieden. Darüber hinaus können aber auch die bisher üblicherweise zwischen den Befestigungsschrauben und der Rippengleitstuhlplatte vorgesehenen Federringe entfallen, so daß deren Überwachung und Austausch während der biologischen Lebensdauer einer Holzschwelle 2 ebenfalls vermieden werden kann.

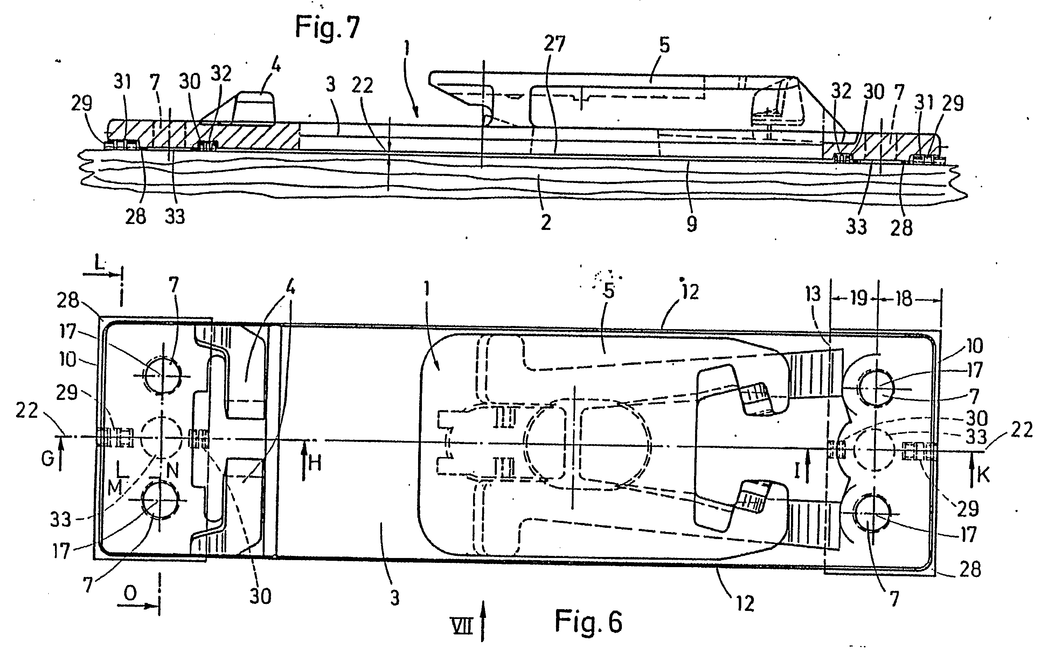

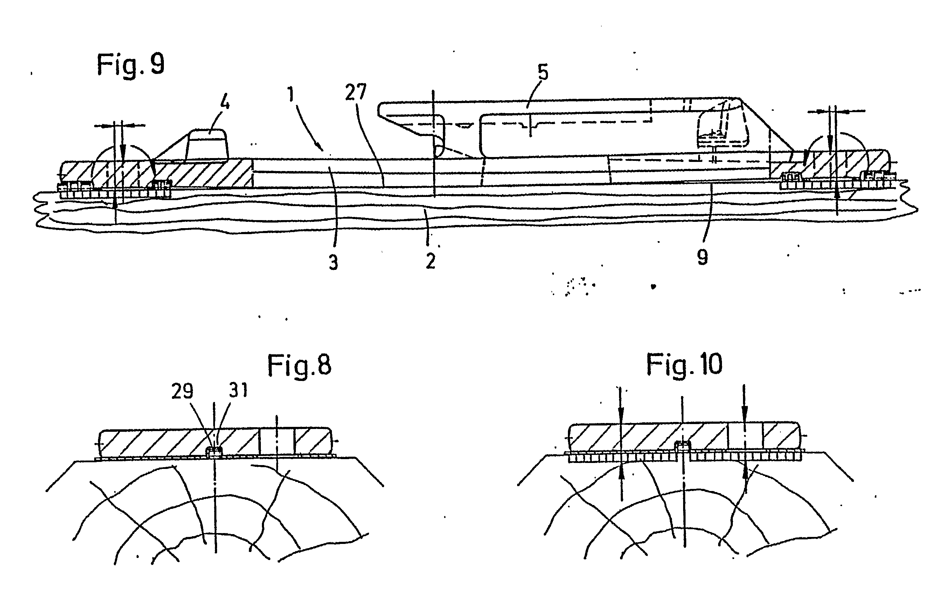

Die in den Fig. 6 bis 10 dargestellte Rippengleitstuhlplatte 1 hat grundsätzlich die gleiche Wirkungsweise wie diejenige nach den Fig. 1 bis 5. Unterschiedlich ist dort lediglich, daß die Podeste 8 an der Unterseite 27 der Unterlagsplatte 3 nicht einstückig angegossen sind, sondern vielmehr durch einstückige Beilageteile 28 aus Blech oder Kunststoff gebildet werden, die an der Unterlagsplatte 3 verklemmt oder verrastet werden können. Auch die als Podeste dienenden Beilageteile 28 haben dabei eine Dicke, die zwischen 1 mm und 2 mm liegt und sie sind darüber hinaus so gestaltet, daß ihre äußere Querkante 10 von der Längsachse der Durchgangslöcher 7 einen Abstand 18 aufweist, welcher größer ist als der Abstand 19, den ihre innere Querkante 13 von der Achse 17 der Durchgangslöcher 7 hat.

Abweichend von den Podesten 8 beim Ausführungsbeispiel nach den Fig. 1 bis 5 sind die Beilageteile beim Ausführungsbeispiel nach den Fig. 6 bis 10 so ausgeführt, daß sie ununterbrochen über die gesamte Breite der Unterlagsplatte durchgehen.

Etwa auf halber Länge sind die Beilageteile 28 mit aus ihrer Ebene ausgeklinkten und hakenartig hochgestellten Klemm- und Rastzungen 29 versehen, die mit in der Plattenunterseite 27 vorgesehenen Vertiefungen in Ausricht- und Halteeingriff gelangen.

In dem Bereich zwischen den Klemm- und Rastzungen 29 und 30 weist darüber hinaus jedes der Beilageteile 28 noch einen zusätzlichen Ausschnitt 33, bspw. in Form eines runden Loches auf, das etwas größer als die Durchgangslöcher 7 in der Unterlagsplatte 3 ausgeführt werden kann. Zur Benutzung der Beilageteile 28 als Podeste eignen sich Rippengleitstuhlplatten 1 herkömmlicher Bauart praktisch in unveränderter Form. Gegebenenfalls ist es lediglich notwendig, in der Unterseite 27 die Vertiefungen 31 und 32 einzuarbeiten, welche die Klemm- und Rastzungen 29 und 30 der Beilageteile 28 aufnehmen können. Denkbar wäre es allerdings auch, in Verbindung mit den herkömmlichen Rippengleitstuhlplatten 1 Beilageteile 28 ohne Klemm- und Rastzungen 29 und 30 zu benutzen und deren Halteverbindung mit der Unterlagsplatte 3 durch einen Haftkleber oder dergleichen zu bewirken.

Selbstverständlich beschränkt sich die Ausstattung mit Podesten 8 und Beilageteilen 28 nicht auf die als Ausführungsbeispiele in der Zeichnung dargestellten Rippengleitstuhl platten. Vielmehr kann sie auch bei Rippenplatten jeder anderen Bauart zum Einsatz gelangen, bspw. bei den üblichen K-Rippenplatten, bei Stützbockplatten oder dergleichen.

## Patentansprüche

1. Unterlagsplatte, insbesondere Rippenplatte, für die Befestigung der Schienen von Eisenbahngleisen und -weichen auf Holzschwellen; die jeweils in der Nähe ihrer Enden mittels in Durchgangslöcher eingreifender Schrauben oder dergleichen mit der Schwelle verbunden ist, während im Plattenbereich zwischen den schwellenseitigen Befestigungsstellen jeweils der Schienenfuß abgestützt ist, **dadurch gekennzeichnet,** daß an der Plattenunterseite (27) lediglich im Bereich der Durchgangslöcher (7) für die Befestigungsschrauben aus der Plattenebene vorspringende Podeste (8 bzw. 28) vorgesehen oder ausgebildet sind.

2. Unterlagsplatte nach Anspruch 1, **dadurch gekennzeichnet,** daß die Podeste (8) einstückig an die Plattenunterseite (27) angeformt sind (Fig. 2 bis 5).

3. Unterlagsplatte nach Anspruch 1, **dadurch gekennzeichnet,** daß die Podeste durch Beilageteile (28) aus Blech oder Kunststoff gebildet und an der Plattenunterseite (27) verklemmt, verklebt oder verrastet sind (29 bis 32; Fig. 6 bis 10).

4. Unterlagsplatte nach einem der Ansprüche 1 bis 3, **dadurch gekennzeichnet,** daß die Podeste (8 bzw. 28) eine Dicke von etwa 1 bis 2 mm, vorzugsweise 1,5 mm, aufweisen.

5. Unterlagsplatte nach einem der Ansprüche 1 und 2, **dadurch gekennzeichnet,**

daß jedem Durchgangsloch (7) ein eigener Podestbereich (8) zugeordnet ist, daß die Podestbereiche durch eine Nut (16) voneinander getrennt sind und daß dabei der Abstand des Begrenzungsrandes der Podeste (8) vom Begrenzungsrand des zugehörigen Durchgangsloches (7) größer als der Durchmesser des Durchgangsloches (7) gewählt ist.

6. Unterlagsplatte nach einem der Anspreüche 1 bis 5,
**dadurch gekennzeichnet,**
daß der Abstand (18) der Längsachse (17) des Durchgangsloches (7) bzw. der Abstand des Durchgangsloch-Begrenzungsrandes von dem benachbarten Platten-Ende (6) und der zugehörigen äußeren Podest-Querkante (10) größer bemessen ist als der Abstand (19) der Achse (17) des Durchgangsloches (7) bzw. als der Abstand des Durchgangsloch-Begrenzungsrandes von der inneren Querkante (13) des Podestes (8 bzw. 28).

7. Unterlagsplatte nach einem der Ansprüche 1 bis 6,
**dadurch gekennzeichnet,**
daß das Abstandsverhältnis der äußeren Podest-Querkante (10) und der inneren Podest-Querkante (13) gegenüber der Achse (17) des Durchgangsloches (7) bzw. gegenüber dem Durchgangsloch-Begrenzungsrand bei etwa 1,2 : 1 liegt.

8. Unterlagsplatte nach einem der Ansprüche 1 bis 7,
**dadurch gekennzeichnet,**
daß die Podeste (8 bzw. 28) eine polygonförmige, z.B. etwa rechteckige oder quadratische, Umrißform aufweisen.

9. Unterlagsplatte nach einem der Ansprüche 1, 2 sowie 4 bis 8,
**dadurch gekennzeichnet,**
daß in der Plattenunterseite (27) jeweils an die inneren Podest-Längskanten (13) und die inneren Podest-Querkanten (14) angrenzende Nuten (15 und 16) vorgesehen sind.

10. Unterlagsplatte nach einem der Ansprüche 1, 3 und 4 bis 8,
**dadurch gekennzeichnet,**
daß die die Podeste bildenden Beilageteile (28) aus ihrer Ebene ausgeklinkte und hakenartig hochgestellte Klemm-und Rastzungen (29, 30) aufweisen, die mit in der Plattenunterseite (27) vorgesehenen Vertiefungen (31, 32) in Ausricht- und Halteingriff bringbar sind (Fig. 6 bis 10).

11. Unterlagsplatte nach Anspruch 10,
**dadurch gekennzeichnet,**
daß die die Podeste bildenden Beilageteile (28) sich über die ganze Plattenbreite erstrecken, wobei die Klemm- oder Rastzungen (29, 30) sowie ein zusätzlich zwischen diesen vorgesehener Ausschnitt (33) sich etwa auf halber Länge der Beilageteile (28) befinden.

12. Unterlagsplatte, insbesondere nach Anspruch 5,
**dadurch gekennzeichnet,**
daß die Ausnehmung (16) auf der Längsmitte (22-22) eine Anordnung und/oder Ausbildung hat, bei der aus der QuerEntfernung der Bohrungen kein Moment - weder konkav noch konvex - auftritt und die Resultierende der Schwellenschraubenachsen sind in der gleichen Ebene befinden wie die beiden Resultierenden aus den beiden Flächenbelastungsseiten.

13. Unterlagsplatte nach einem der Ansprüche 1 bis 12,
**dadurch gekennzeichnet,**
daß anstelle eines Gleitstückes (5) ein Stützbock für das Abstützen eines Radlenkerprofils im Herzstück einer Weiche vorgesehen ist.

## Revendications

1. Selle de rail, notamment nervurée pour la fixation des rails et aiguillages de chemins de fer sur des traverses en bois, qui est reliée, chaque fois à proximité de ses extrémités, à la traverse à l'aide de vis ou analogues pénétrant dans des perçages, tandis que la semelle de rail est chaque fois appuyée dans la zone de selle comprise entre les points de fixation à la traverse, caractérisée en ce que sur la face inférieurs de la selle (27), uniquement dans la zone des perçages (7) destinés aux vis de fixation, des socles (8 ou 28) faisant saillie sur le plan de la selle sont prévus ou formés, la distance (18) entre l'axe longitudinal (17) du perçage ou le bord limitant le perçage et l'extrémité adjacente (6) de la selle et l'arête transversale extérieure correspondante (10) du socle est plus grande que la distance (19) entre l'axe (17) du perçage (7) ou le bord limitant le perçage et l'arête transversale intérieure (13) du socle (8 ou 28).

2. Selle de rail selon la revendication 1, caractérisée en ce que les socles (8) sont formés d'une seule pièce à la face inférieure (27) (Figures 2 à 5).

3. Selle de rail selon la revendication 1, caractérisée en ce que les socles consistent en des pièces rapportées (28) en feuille métallique ou en matière synthétique et sont serrés, collés ou encliquetés (29 à 32; figures 6 à 10) sur la face inférieure (27).

4. Selle de rail selon l'une quelconque des revendications 1 à 3, caractérisée en ce que les socles (8 ou 28) présentent une épaisseur d'environ 1 à 2 mm, de préférence 1,5 mm.

5. Selle de rail selon l'une quelconque des revendications 1 et 2, caractérisée en ce qu'une zone de socle propre est attribuée à chaque perçage (7), en ce que les zones de socle sont séparées l'une de l'autre par une rainure (16) et en ce que la distance entre le bord des socles (8) et le bord du perçage correspondant (7) est supérieure au diamètre du perçage (7).

6. Selle de rail selon l'une quelconque des revendications 1 à 5, caractérisée en ce que le rapport des distances entre l'arête transversale extérieurs (10) du socle et l'arête transversale intérieure (13) du socle et l'axe (17) du perçage (7) ou le bord délimitant le perçage est d'environ 1,2:1.

7. Selle de rail selon l'une quelconque des revendications 1 à 6, caractérisée en ce que les socles (8 ou 28) affectent une forme extérieure polygonale, par exemple approximativement rectangulaire ou carrée.

8. Selle de rail selon l'une quelconque des revendications 1,2, ainsi que 4 à 7, caractérisée en ce que des rainures (15 et 16) pratiquées dans la face inférieurs de la selle sont prévues à proximité chaque fois des arêtes longitudinales intérieures (13) et des arêtes transversales intérieures (14) du socle.

9. Selle de rail selon l'une quelconque des revendications 1, 3 et 4 à 7, caractérisée en ce que les pièces rapportées (28) formant les socles présentent des languettes de serrage et d'encliquetage (29, 30) pliées et redressées en dehors de leur plan à la manière d'un crochet, qui sont adaptables (figures 6 à 10) à des évidements (31, 32) pratiqués dans la face inférieure (27) de la selle en vue de l'orientation et de la fixation.

10. Selle de rail selon la revendication 9, caractérisée en ce que les pièces rapportées (25) formant les socles s'étendent sur toute la largeur de selle, les languettes de serrage et d'encliquetage (29, 30) ainsi qu'une découpe (33) prévue en plus entre celles-ci se trouvant approximativement à demi longueur des pièces rapportées (28).

11. Selle de rail selon l'une quelconque des revendications précédentes, notamment la revendication 5, caractérisée en ce que l'évidement (16) sur l'axe longitudinal médian (22-22) présente une disposition et/ou une forme telle que la distance transversale entre perçages ne peut générer aucun moment – ni concave, ni convexe – et que la résultante des axes des vis de traverse se trouve dans le même plan que les deux résultantes des deux côtés sollicités en surface.

12. Selle de rail selon l'une quelconque des revendications 1 à 11, caractérisée en ce qu'un appui est prévu dans la partie centrale d'un aiguillage pour l'appui d'un profilé de contre-rail, au lieu du siège de glissement (5).

**Claims**

1. Sole plate, more especially a ribbed plate, for the fixing of the rails of railway tracks and points on wood sleepers, which plate is connected to the sleeper in the vicinity of its ends and by means of screw-bolts or the like engaging in holes, while the rail base in each case is connected in the plate-region between the fixing points on the sleeper side, wherein platforms (8 or 28) are provided or formed on the underside (27) of the plate, only in the region of the through holes (7) for the fixing screw bolts, the spacing (18) of the longitudinal axis (17) of the through hole (7) or respectively the spacing of the limiting rim of the through hole from the adjoining end (6) of the plate and the associated outer platform transverse edge (10) is made larger than the spacing (19) of the axis (17) of the through hole (7) or than the spacing of the rim of the through hole from the inner transverse edge (13) of the platform (8 or 28).

2. Sole plate according to claim 1, wherein the platforms (8) are formed in one piece on the underside (27) of the plate (Figs. 2 to 5).

3. Sole plate according to claim 1, wherein the platforms are formed by spacer parts (28) of sheet metal or synthetic plastics and are clamped, stuck or secured at (29 to 32) on the underside (27) of the plates (Figs. 6 to 10).

4. Sole plate according to one of the claims 1 to 3, wherein the platforms (8 or 28) have a thickness of about 1 to 2 mm., advantageously 1.5 mm.

5. Sole plate according to one of the claims 1 and 2, wherein each through hole (7) has its own platform region (8) associated therewith, that the platform regions are separated by a groove (16) and that, as a result, the distance of the rims of the platforms (8) from the rim of the associated through hole (7) is chosen to be larger than the diameter of the through hole (7).

6. Sole plate according to one of the claims 1 to 5, wherein the spacing ratio of the outer platform transverse edge (10) and of the inner platform transverse edge (13) with respect to the axis (17) of the through hole (7) or respectively to the rim of the through hole is in the region of 1.2:1.

7. Sole plate according to one of the claims 1 to 6, wherein the platforms (8 or 28) have a contour form which is polygonal, for example, approximately rectangular or square.

8. Sole plate according to one of the claims 1, 2 and also 4 to 7, wherein grooves (15 and 16) adjoining the inner platform longitudinal edges (13) and the inner platform longitudinal edges (14) are provided in the underside (27) of the plates.

9. Sole plate according to one of the claims 1, 3 and 4 to 7, wherein the shim-like parts (28) forming the platforms comprise clamping and latching tongues (29, 30) released from their plane and raised in hook-like fashion, which tongues are able to be brought into the aligning and holding engagement by means of depressions (31, 32) provided in the undersides (27) of the plates (Figs. 6 to 10).

10. Sole plate according to claim 9, wherein the spacer parts (28) forming the platforms are extended over the full width of the plates, the clamping or latching tongues (29, 30) and also 3 cut-out (33) additionally provided between these latter are situated approximately half-way between the spacer parts (28).

11. Sole plate according to one of the preceding claims, more especially claim 5, wherein the groove (16) at the mid-point (22-22) of the length has an arrangement and/or construction with which, arising from the transverse spacing of the bores, there is no moment – neither concave nor convex – and the resultants of the sleeper bolt axes are disposed in the same plane as the two resultants from the two surface-loading sides.

12. Sole plate according to one of the claims 1 to 11, wherein a supporting block is provided, instead of a sliding element (5), for the support of a wheel-guiding section member in the frog of a switch point.

Fig.2

EP 0 275 604 B1

Fig. 1

Fig. 4

Fig. 5

Fig. 3

Fig. 7

EP 0 275 604 B1

Fig. 6

Fig. 9

4 1 27 5

3 2 9

Fig. 8

29 31

Fig. 10

EP 0 275 604 B1